# EUROPEAN PATENT APPLICATION

(11) **EP 4 385 987 A1**
(43) Date of publication of application: **19.06.2024**
(21) Application number: 22855220.4
(22) Date of filing: 25.07.2022
(51) Int. Cl.: C07D 403/06, A61K 31/496

(54) **IMPURITY IN PLINABULIN OR PREPARATION THEREOF, AND USE THEREOF**

(30) Priority: 13.08.2021 CN 202110932963
(71) Applicant: Beyondspring Pharmaceuticals, Inc, Florham Park, NJ 07932 (US)
(72) Inventor: HE, Chengjiang, Dalian, Liaoning 116620 (CN); ZHANG, Danyang, Dalian, Liaoning 116620 (CN); MAO, Jianmin, Dalian, Liaoning 116620 (CN); DU, Lihua, Dalian, Liaoning 116620 (CN); HUANG, Lan, Dalian, Liaoning 116620 (CN)
(74) Representative: dompatent von Kreisler Selting Werner - Partnerschaft von Patent- und Rechtsanwälten mbB
(86) International application number: PCT/CN2022/107702
(87) International publication number: WO 2023/016237

(57) **Abstract**

The present invention relates to an impurity in plinabulin or a preparation thereof, and a use thereof. The impurity has the structure as shown in formula I, and can be obtained by means of the oxidative degradation of plinabulin under neutral or acidic conditions, having great significance in the quality control of plinabulin or a preparation thereof.

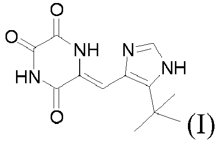

## Description

### TECHNICAL FIELD

The invention belongs to the field of medicine, and specifically relates to an impurity in Plinabulin or preparation thereof, and use thereof.

### TECHNCIAL BACKGROUND

Plinabulin, ((3Z,6Z)-3-[(5-tert-butyl-1H-imidazol-4-yl)methylene]-6-(benzylidene)-2,5-piperaz inedi Ketone), is a synthetic analogue of the diketopiperazine phenylahistin (benzyltrimethylammonium chloride) found in marine and terrestrial Aspergillus species and the structure thereof is as follows:

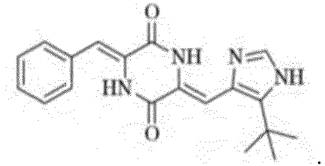

Plinabulin is structurally distinct from colchicine and combretastatin-like analogues thereof (e.g., combretastatin phosphate) and binds to the colchicine binding site on the tubulin monomer or nearby sites. Previous studies have shown that Plinabulin at low concentrations induces tubulin depolymerization and monolayer permeability in vascular endothelial cells compared with colchicine and indicate that Plinabulin induces apoptosis in Jurkat leukemia cells. Plinabulin has demonstrated great pharmacokinetic, pharmacodynamic and safety property as a single therapeutic agent in patients with advanced malignancies (lung, prostate and colon cancer).

Impurity research is an important part of research and development of pharmaceuticals, which runs through the entire process of research and development of pharmaceuticals and directly affects the safety, effectiveness and quality control of drugs. The quality control of drugs is mainly to control the content of active ingredients and related substances. In particular, the content of related substances needs to meet medicinal requirements. The impurities mainly come from the synthesis process and drug degradation. In the process of drug quality testing, high-purity impurities are required to be used as control standards to control the quality of drugs that may contain the impurities. Therefore, it is significantly important to provide an impurity standard of Plinabulin for quality control of Plinabulin or a pharmaceutical composition thereof.

### SUMMARY OF THE INVENTION

The purpose of the present invention is to provide an impurity of Plinabulin and a preparation method thereof, and its use in pharmaceutical quality control.

The first aspect of the invention is to provide a compound of formula I

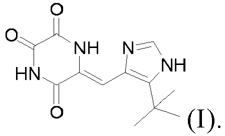

The second aspect of the invention is to provide a composition, wherein the composition comprises i) Plinabulin and ii) the compound of formula I of the first aspect.

In another preferred embodiment, the composition comprises: (a) a safe and therapeutically effective amount of Plinabulin polymorph or a pharmaceutically acceptable salt thereof, and (b) pharmaceutically acceptable carriers, diluents, excipients or combinations thereof.

In another preferred embodiment, the diluent comprises 15-hydroxypolyethylene glycol stearate.

In another preferred embodiment, the diluent comprises propylene glycol.

In another preferred embodiment, the diluent comprises 15-hydroxypolyethylene glycol stearate and propylene glycol.

In another preferred embodiment, the diluent is 15-hydroxypolyethylene glycol stearate and propylene glycol, wherein 15-hydroxypolyethylene glycol stearate is about 40 wt% and propylene glycol is about 60 wt%.

In another preferred example, the mass ratio of Plinabulin to the compound of formula I is 1-10000:10000-1.

In another preferred embodiment, the content of Plinabulin ranges from 0.01% to 99.99%.

The third aspect of the present invention is to provide a pharmaceutical composition, wherein the pharmaceutical composition comprises i') the composition of the second aspect and ii') a pharmaceutically acceptable carrier.

In another preferred embodiment, the pharmaceutical composition may be an injection, a tablet, a capsule, an aerosol, a suppository, a film, a dropping pill, an external liniment, a controlled-release type or slow-release type or nano-formulation.

In another preferred embodiment, a single dose of the composition is from about 1 mg to about 300 mg.

The fourth aspect of the present invention is to provide a method for preparing the compound of formula I of the first aspect, comprising the step: 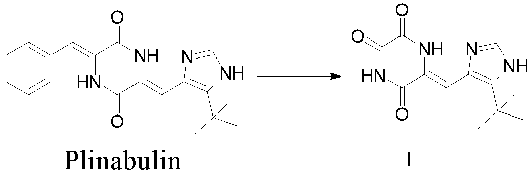
1) In an inert solvent, reacting Plinabulin with an oxidant under a neutral or acidic condition to give the compound of formula I.

In another preferred embodiment, the inert solvent is selected from: ethanol, ethylene glycol, DMSO, DMF, or combinations thereof.

In another preferred embodiment, the acid is HCl, HAc, or a combination thereof.

In another preferred example, the reaction temperature is 40-80 °C, preferably 50-60 °C.

In another preferred embodiment, the molar ratio of acid to Plinabulin is 200:1-20:1, preferably 100:1-15:1.

In another preferred embodiment, the oxidant is selected from: peroxide, oxygen, or a combination thereof.

The fifth aspect of the present invention is to provide use of the compound of formula I of the first aspect, wherein the compound is used as a standard for quality control of Plinabulin API (Active Pharmaceutical Ingredients) or preparation thereof.

In another preferred embodiment, the control is achieved by using high performance liquid chromatography to determine the content of the compound of formula I in Plinabulin API or a preparation thereof that calculated by an area normalization method.

It should be understood that within the scope of the present invention, the above-mentioned technical features of the present invention and the technical features specifically described below (such as embodiments) may be combined with each other to form a new or preferred technical solution, which will not be repeated herein one by one due to the limited contents.

### DESCRIPTION OF THE DRAWINGS

Figure 1 shows HPLC profile of a Plinabulin formulation.
Figure 2 shows HPLC profile of Plinabulin API after acid degradation.
Figure 3 shows the H NMR spectrum of the impurity of Plinabulin.
Figure 4 shows the mass spectrum of the impurity of Plinabulin.

### DETAILED DESCRIPTION OF THE EMBODIMENT

After extensive and in-depth research, the inventors unexpectedly discovered an impurity in Plinabulin preparation for the first time, which is also produced in the acid degradation test of Plinabulin API. The inventors destroyed the Plinabulin API in the presence of oxidants under neutral and acidic conditions, and separated and purified the products to obtain the impurity of Plinabulin. On this basis, the present invention is completed.

### Terms

"RT" refers to retention time;
"RRT" refers to relative retention time;
"API" refers to active pharmaceutical ingredient.

### Impurity of Plinabulin

In the present invention, "impurity of Plinabulin", "NPI-2602" and "compound of formula I" have the same meaning and may be used interchangeably.

The structure of NPI-2602 is as follows

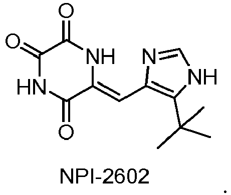

Due to oxidation, Plinabulin will degrade and produce impurities during storage, transportation, and the like, thus affecting the purity of Plinabulin. The impurity in the HPLC spectrum of Plinabulin preparation is at RT-5.002 min (RRT=0.61), by comparing mass spectrometry and retention time in the acid degradation experiment of Plinabulin API, the impurity is also produced.

### Plinabulin composition and administration

The Plinabulin composition of the present invention refers to a composition containing Plinabulin and a compound of formula I. Preferably, the mass ratio of Plinabulin to the compound of formula I is 1-10000:10000-1, preferably, 1:1000 to 1000:1; more preferably, 1:500 to 500:1; even more preferably, 1:100 to 100:1.

Preferably, the content of Plinabulin ranges from 0.01% to 99.99% based on the total weight of the composition; preferably, 0.1% to 99.9%; more preferably, 99.80% to 99.99%; even more preferably, 99.90% to 99.95%.

Preferably, the content of the impurity of Plinabulin ranges from 0.01% to 99.99% based on the total weight of the composition; preferably, 0.1% to 99.9%; more preferably, 0.20% to 0.01%; even more preferably, 0.1 % to 0.05%.

In some embodiments, the compositions may also comprise one or more pharmaceutically acceptable diluents. In some embodiments, a pharmaceutically acceptable diluent may comprise 15-hydroxypolyethylene glycol stearate (kolliphor). In some embodiments, the pharmaceutically acceptable diluent may comprise propylene glycol. In some embodiments, the pharmaceutically acceptable diluent may comprise kolliphor and propylene glycol. In some embodiments, the pharmaceutically acceptable diluent may comprise kolliphor and propylene glycol, wherein kolliphor is about 40 wt% and propylene glycol is about 60 wt%, based on the total weight of the diluent. In some embodiments, the compositions may also comprise one or more other pharmaceutically acceptable excipients.

Standard pharmaceutical formulation techniques may be used to prepare the drug compositions described herein, such as those disclosed in Remington's The Science and Practice of Pharmacy, 21st Edition, Lippincott Williams & Wilkins (2005), which is incorporated herein by reference in its entirety. Accordingly, some embodiments comprise pharmaceutical compositions comprising: (a) a safe and therapeutically effective amount of a Plinabulin polymorph or a pharmaceutically acceptable salt thereof, and (b) a pharmaceutically acceptable carrier, diluent, excipient, or combination thereof.

Other embodiments comprise co-administering the Plinabulin polymorph and the additional therapeutic agent in separate compositions or the same composition. Accordingly, some embodiments comprise a first pharmaceutical composition and a second pharmaceutical composition, wherein the first pharmaceutical composition comprises: (a) a safe and therapeutically effective amount of a Plinabulin polymorph or a pharmaceutically acceptable salt thereof and (b) a pharmaceutically acceptable carrier, diluent, excipient, or combination thereof; and the second pharmaceutical composition comprises: (a) a safe and therapeutically effective amount of an additional therapeutic agent and (b) a pharmaceutically acceptable carrier, diluent, excipient, or combination thereof. Some embodiments comprise pharmaceutical compositions comprising: (a) a safe and therapeutically effective amount of a Plinabulin polymorph or a pharmaceutically acceptable salt thereof; (b) a safe and therapeutically effective amount of an additional therapeutic agent; and (c) pharmaceutically acceptable carrier, diluent, excipient, or combination thereof.

The formulation of the pharmaceutical composition of the present invention comprises (but not limited to): an injection, a tablet, a capsule, an aerosol, a suppository, a film, a dropping pill, an external liniment, a controlled-release type or slow-release type or nano-formulation.

The administration of the pharmaceutical composition described herein may be via any acceptable administration (for similar purposes) including, but not limited to, oral, sublingual, buccal, subcutaneous, intravenous, intranasal, topical, transdermal, intradermal, intraperitoneal, intramuscular, intrapulmonary, vaginal, rectal or intraocular administration. Oral and parenteral administration is commonly used to treat the indications that are the subject of preferred embodiments.

The term "pharmaceutically acceptable carrier" or "pharmaceutically acceptable excipient" comprises any and all solvents, dispersion media, coatings, antibacterial and antifungal agents, isotonic and absorption delaying agents, and the like. The use of such media and reagents for pharmaceutically active substances is well known in the art. In addition, a variety of adjuvants may be included, for example, that are commonly used in the art. Considerations for the inclusion of various components in pharmaceutical compositions are described in, for example, Gilman et al. (Eds.) (1990); Goodman and Gilman's: The Pharmacological Basis of Therapeutics, 8th Edition, Pergamon Press, which is incorporated by reference in its entirety.

Some examples of substances that may be used as pharmaceutically acceptable carriers or components thereof are: sugars, such as lactose, glucose and sucrose; starches, such as corn starch and potato starch; cellulose and derivatives thereof, such as carboxymethyl cellulose sodium, ethylcellulose, and methylcellulose; powdered tragacanth; malt; gelatin; talc; solid lubricants, such as stearic acid and magnesium stearate; calcium sulfate; vegetable oils, such as peanut oil, cottonseed oil, sesame oil, olive oil, corn oil and cocoa butter; polyols such as propylene glycol, glycerin, sorbitol, mannitol and polyethylene glycol; alginic acid; emulsifiers such as TWEENS; wetting agents such as sodium lauryl sulfate; colorants; flavoring agents; tablet-making agents; stabilizers; antioxidants; preservatives; pyrogen-free water; isotonic saline; and phosphate buffered solutions.

Compositions described herein are preferably provided in unit dosage form. As used herein, a "unit dosage form" is a composition containing an amount of compound or composition suitable for administration in a single dose to an animal, preferably a mammalian subject, in accordance with good medical practice. However, the formulation of a single dosage form or unit dosage form does not mean that the dosage form is to be administered once daily or once per course of treatment. It is contemplated that the dosage form may be administered once, twice, three or more times daily and may be administered as an infusion over a period of time (e.g., about 30 minutes to about 2-6 hours) or as a continuous infusion and may be administered during a course of treatment administered more than once during the period, but a single administration is not specifically excluded. Those skilled in the art will recognize that the formulation does not take into account the entire course of treatment and such decisions should be left to those skilled in the therapeutic art rather than the formulation art.

Useful compositions as described above may be used in any of a variety of suitable formulations for various routes of administration, for example, for oral, sublingual, buccal, nasal, rectal, topical (including transdermal and intradermal)), ocular, intracerebral, intracranial, intrathecal, intraarterial, intravenous, intramuscular or other parent routes of administration. Those skilled in the art will understand that oral and nasal compositions comprise compositions administered by inhalation and prepared using appropriate techniques. Depending on the particular route of administration desired, a variety of pharmaceutically acceptable carriers well known in the art may be used. Pharmaceutically acceptable carriers comprise, for example, solid or liquid fillers, diluents, co-solvents, surfactants and encapsulating substances. Optionally pharmaceutically active materials may be comprised which do not substantially interfere with the activity of the compound or composition. The amount of carrier used in combination with the compound or composition is sufficient to provide the actual amount of material per unit dose of compound for administration. Techniques and compositions for preparing dosage forms suitable for use in the methods of the present application are described in the following references, both of which are incorporated herein by reference: Modern Pharmaceutics, 4th Edition, Chapter 9 and Chapter 10 (Banker and Rhodes, eds., 2002); Lieberman et al., Pharmaceutical Dosage Forms: Tablets (1989); and Ansel, Introduction to Pharmaceutical Dosage Forms, 8th Edition, (2004).

A variety of oral dosage forms may be used, including, for example, solid forms such as tablet, capsule (eg, liquid gel capsule and solid gel capsule), granule, and bulk powder. The tablet may be compressed tablet, molded tablet, sugar-coated, film-coated or multi-compressed tablet, containing suitable binder, lubricant, diluent, disintegrating agent, coloring agent, flavoring agent, flow inducer and melting agent. The liquid oral dosage forms comprise aqueous solution, emulsion, suspension, solution and/or suspensions reconstituted from non-effervescent granules and effervescent preparations reconstituted from effervescent granules, containing appropriate solvents and preservatives, emulsifiers, suspending agents, diluents, sweeteners, melting agents, colorants and flavoring agents.

The pharmaceutically acceptable carriers suitable for unit dosage forms for oral administration are well known in the art. Tablets usually comprise conventional pharmaceutically compatible adjuvants as inert diluents, such as: calcium carbonate, sodium carbonate, mannitol, lactose and cellulose; binders, such as starch, gelatin and sucrose; disintegrating agents, such as starch, alginic acid and croscarmellose; lubricants such as magnesium stearate, stearic acid and talc. Glidants such as silica may be used to improve the flow characteristics of the powder mixture. For appearance, colorants such as FD&C dyes may be added. For chewable tablets, sweetening and flavoring agents such as aspartame, saccharin, menthol, peppermint, sucrose and fruit flavors are useful adjuvants. Capsules typically comprise one or more solid diluents disclosed above. The selection of carrier components depends on secondary considerations such as taste, cost and storage stability, which are not critical factors and may be readily accomplished by those skilled in the art.

Oral compositions also comprise liquid solutions, emulsions, suspensions, and the like. Pharmaceutically acceptable carriers suitable for use in preparing such compositions are well known in the art. Typical carrier ingredients for syrups, elixirs, emulsions and suspensions comprise ethanol, glycerol, propylene glycol, polyethylene glycol, liquid sucrose, sorbitol and water. For suspensions, typical suspending agents comprise methylcellulose, sodium carboxymethylcellulose, AVICELRC-591, tragacanth gum, and sodium alginate; typical wetting agents comprise lecithin and polysorbate 80; and typical preservatives comprise methyl paraben and sodium benzoate. Oral liquid compositions may also contain one or more of the ingredients disclosed above, such as sweetening agents, flavoring agents and coloring agents.

Such compositions may also be coated by conventional methods, typically with a pH-dependent or time-dependent coating, such that the composition is released in the gastrointestinal tract in proximity to the desired local administration, or at different times to prolong desired activity. Such formulations typically comprise, but are not limited to, one or more of cellulose acetate phthalate, polyethylene acetate phthalate, hydroxypropyl methylcellulose phthalate, ethyl cellulose, Eudragit coating, wax and shellac.

The compositions described herein may optionally comprise other pharmaceutical actives.

Other compositions for systemic delivery of the compounds of the present application comprise sublingual, buccal, and nasal formulations. Such compositions typically contain one or more of the following group: soluble filler materials such as sucrose, sorbitol, and mannitol; and binders such as gum arabic, microcrystalline cellulose, carboxymethylcellulose, and hydroxypropyl Methylcellulose. Glidants, lubricants, sweeteners, colorants, antioxidants and flavoring agents disclosed above may also be comprised.

Liquid compositions for topical ophthalmic use are formulated so that they may be applied topically to the eye. Comfort may be maximized as much as possible, but sometimes considering formulation (e.g., drug stability) may not allow optimal comfort may not be achieved. Where comfort cannot be maximized, the liquid may be formulated so that it is tolerable to the patient for topical ophthalmic use. Additionally, ophthalmologically acceptable liquids may be packaged for single use or contain preservatives to prevent contamination during multiple uses.

For ocular applications, a saline solution is often used as the primary carrier to prepare the solution or drug. Ophthalmic solutions may preferably be maintained at a comfortable pH using an appropriate buffer system. The formulations may also contain conventional, pharmaceutically acceptable preservatives, stabilizers and surfactants.

Preservatives that may be used in the pharmaceutical compositions disclosed herein comprise, but are not limited to, benzalkonium chloride, PHMB, chlorobutanol, thimerosal, phenylmercuric acetate, and phenylmercuric nitrate. Useful surfactants are, for example, Tween 80. Likewise, a variety of useful carriers may be used in the ophthalmic formulations disclosed herein. These carriers comprise, but are not limited to, polyvinyl alcohol, povidone, hydroxypropyl methylcellulose, poloxamer, carboxymethyl cellulose, hydroxyethyl cellulose and purified water.

Tension adjusters may be added as needed or as convenient. Tension adjusters comprises, but is not limited to, salts (especially sodium chloride, potassium chloride), mannitol and glycerin, or any other suitable ophthalmologically acceptable tonicity adjusting agent.

Various buffers and means for adjusting the pH may be used as long as the resulting formulation is ophthalmologically acceptable. For many compositions, pH will be 4 to 9. Thus, buffers comprise acetate buffer, citrate buffer, phosphate buffer and borate buffer. Acids or bases may be used to adjust the pH of these formulations, as desired.

Ophthalmologically acceptable antioxidants comprise, but are not limited to, sodium metabisulfite, sodium thiosulfate, acetylcysteine, butylated hydroxyanisole, and butylated hydroxytoluene.

Other excipient components that may be comprised in ophthalmic formulations are chelating agents. A useful chelating agent is disodium ethylenediaminetetraacetic acid (EDTA), but other chelating agents may be used instead or in combination with EDTA.

For topical use, creams, ointments, gels, solutions or suspensions, and the like containing the compositions disclosed herein are used. Typically topical formulations may consist of a pharmaceutical carrier, co-solvents, emulsifiers, penetration enhancers, preservative systems and emollients.

For injections, administration may be intravenous, and specifically, the compositions described herein may be dissolved or dispersed in a pharmaceutically acceptable diluent, such as physiological saline or dextrose solution. Suitable excipients may be comprised to achieve the desired pH, including, but not limited to, NaOH, sodium carbonate, sodium acetate, HCl, and citric acid. In various embodiments, the pH of the final composition is from 2 to 8, or preferably from 4 to 7. Antioxidant excipients may comprise sodium bisulfite, sodium acetonate bisulfite, sodium formaldehyde sulfoxylate, thiourea, and EDTA. Other non-limiting examples of suitable excipients present in the final intravenous composition may comprise sodium or potassium phosphate, citric acid, tartaric acid, gelatin, and carbohydrates (eg, dextrose, mannitol, and dextran). Additional acceptable excipients are described in Powell et al., Compendium of Excipients for Parenteral Formulations, PDA J Pharm Sci and Tech 1998, 52 238-311 and Nema et al., Excipients and Their Role in Approved Injectable Products: Current Usage and Future Directions, PDA J Pharm Sci and Tech 2011, 65 287-332, both of which are incorporated by reference in their entirety. Antimicrobial agents may also be comprised to provide bacteriostatic or fungistatic solutions, including, but not limited to, phenylmercuric nitrate, thimerosal, benzethonium chloride, benzalkonium chloride, phenol, cresol, and chlorobutanol.

Compositions for intravenous administration may be provided to the caregiver in the form of one or more solids that are reconstituted with a suitable diluent (e.g., sterile water, physiological saline, or aqueous dextrose solution) immediately prior to administration. In other embodiments, the compositions are provided in the form of solutions ready for parenteral administration. In other embodiments, the composition is provided as a further diluted solution prior to administration. In embodiments comprising administration of a compound described herein in combination with an additional agent, the combination may be provided to the caregiver as a mixture, or the caregiver may mix the two agents prior to administration, or the two agents may be administered separately.

In some embodiments, a single dose of a composition or other therapeutic agent may be from about 5 mg/m² to about 150 mg/m² body surface area, from about 5 mg/m² to about 100 mg/m² body surface area, from about 10 mg/m² to about 100 mg/m² body surface area, about 10 mg/m² to about 80 mg/m² body surface area, about 10 mg/m² to about 50 mg/m² body surface area, about 10 mg/m² to about 40 mg/m² body surface area, about 10 mg/m² to about 30 mg/m² body surface area, about 13.5 mg/m² to about 100 mg/m² body surface area, about 13.5 mg/m² to about 80 mg/m² body surface area, about 13.5 mg/m² to about 50 mg/m² body surface area, about 13.5 mg/m² to about 40 mg/m² body surface area, about 13.5 mg/m² to about 30 mg/m² body surface area, about 15 mg/m² to about 80 mg/m² body surface area, about 15 mg/m² to about 50 mg/m² body surface area, or about 15 mg/m² to about 30 mg/m² body surface area. In some embodiments, a single dose of a composition or other therapeutic agent may be from about 13.5 mg/m² to about 30 mg/m² body surface area. In some embodiments, a single dose of a composition or other therapeutic agent may be about 5 mg/m², about 10 mg/m², about 12.5 mg/m², about 13.5 mg/m², about 15 mg/m², about 17.5 mg/m², about 20 mg/m², about 22.5 mg/m², about 25 mg/m², about 27.5 mg/m², about 30 mg/m², about 40 mg/m², about 50 mg/m², About 60 mg/m², about 70 mg/m², about 80 mg/m², about 90 mg/m² or about 100 mg/m² body surface area.

In some embodiments, a single dose (dosage of a one-time medication) of a composition or other therapeutic agent may range from about 1 mg to about 300 mg, from about 5 mg to about 200 mg, from about 7.5 mg to about 200 mg, from about 10 mg to about 100 mg, from about 15 mg to about 100 mg, about 20 mg to about 100 mg, about 30 mg to about 100 mg, about 40 mg to about 100 mg, about 10 mg to about 80 mg, about 15 mg to about 80 mg, about 20 mg to about 80 mg, about 30 mg to about 80 mg, about 40 mg to about 80 mg, about 10 mg to about 60 mg, about 15 mg to about 60 mg, about 20 mg to about 60 mg, about 30 mg to about 60 mg, or about 40 mg to about 60 mg. In some embodiments, a single dose of a composition or other therapeutic agent may be from about 20 mg to about 60 mg, from about 27 mg to about 60 mg, from about 20 mg to about 45 mg, or from about 27 mg to about 45 mg. In some embodiments, a single dose of a composition or other therapeutic agent may be about 1 mg, 2 mg, 3 mg, 4 mg, 5 mg, about 10 mg, about 12.5 mg, about 13.5 mg, about 15 mg, about 17.5 mg, about 20 mg, about 22.5 mg, about 25 mg, about 27 mg, about 30 mg, about 40 mg, about 50 mg, about 60 mg, about 70 mg, about 80 mg, about 90 mg, about 100 mg, about 125 mg, about 150 mg, or about 200 mg.

The administration period may be a multi-week treatment cycle as long as the tumor remains controllable and the regimen is clinically acceptable. In some embodiments, a single dose of the composition or other therapeutic agent may be administered once a week, and preferably, once on each of Day 1 and Day 8 of a three-week (21-day) treatment cycle or on administer on Day 1 of a three-week (21-day) treatment cycle. In some embodiments, a single dose of a composition or other therapeutic agent may be administered once a week, twice a week, three times a week, four times a week, five times a week, six times a week, or administered daily during one, two, three, four, or five-week treatment cycles. The administration may be performed on the same or different days of each week in the treatment cycle.

Treatment cycles may be repeated as long as the regimen is clinically acceptable. In some embodiments, the treatment cycle is repeated n times, where n is an integer from 2 to 30. In some embodiments, n is 2, 3, 4, 5, 6, 7, 8, 9, or 10. In some embodiments, a new treatment cycle may be performed immediately upon completion of a previous treatment cycle. In some embodiments, a new treatment cycle may be performed some time after completion of a previous treatment cycle.

In some embodiments, the compositions described herein may be used in combination with other therapeutic agents. In some embodiments, the compositions described herein may be administered or used in combination with other treatments, such as chemotherapy, radiation, and biological therapies.

### Process for preparing of impurity of Plinabulin

The Plinabulin API is destroyed by acid, and the destroyed API is tested using the analysis method of Plinabulin API-related substances, and the impurity at RRT 0.61 is found, and the impurity is separated and purified.

Preferably, in the present invention, the impurity of Plinabulin is prepared by the following process

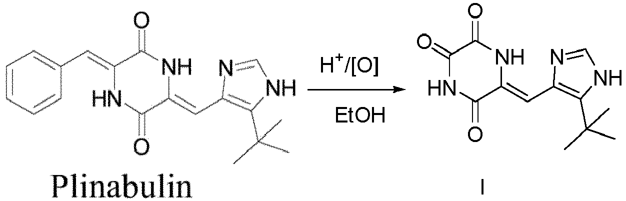

In inert solvents (such as ethanol, DMSO, DMF, and the like), Plinabulin reacts with an oxidant (for example, peroxides such as H₂O₂) in the presence of an acid (such as HCl, HAc, and the like) to give the compound of formula I and benzaldehyde.

The main advantages of the present invention are:
(1) impurity I of Plinabulin is discovered for the first time;
(2) it is of great significance to the quality control of the product of Plinabulin.

The present invention is further illustrated below with reference to specific examples. It should be understood that these examples are merely illustrative of the invention and are not intended to limit the scope of the invention. The experimental methods with no specific conditions described in the following examples are generally performed under the conventional conditions, or according to the manufactures' instructions. Unless otherwise stated, percentages and parts are percentages by weight and parts by weight.

The experimental materials and reagents used in the following examples may be obtained from commercial sources unless otherwise specified.

### Examples

### Example 1 Study on Impurities in Plinabulin Formulations

Plinabulin formulations were analyzed using HPLC. The analysis conditions were as follows:

| | | | |
|---|---|---|---|
| Column: | ACE C18, 150 4.6 mm, 5 m, 100Å | | |
| Mobile phase A: | A) 10 mM sodium phosphate buffer solution | | |
| Mobile phase B: | B) ACN | | |
| Flow rate: | 1.2 mL/min | | |
| Column temperature: | 40°C | | |
| Injection temperature: | 20°C | | |
| Injection volume: | 10 µL | | |
| Detection wavelength: | 330 nm | | |
| Reaction time of Plinabulin: | 12 min | | |
| Operation time: | 20 min | | |
| | Time (min) | %A | %B |
| Gradients: | 0 | 90 | 10 |
| | 4.0 | 60 | 40 |
| | 15.0 | 55 | 45 |
| | 16.0 | 20 | 80 |
| | 17.0 | 20 | 80 |
| | 17.1 | 90 | 10 |
| | 20.0 | 90 | 10 |

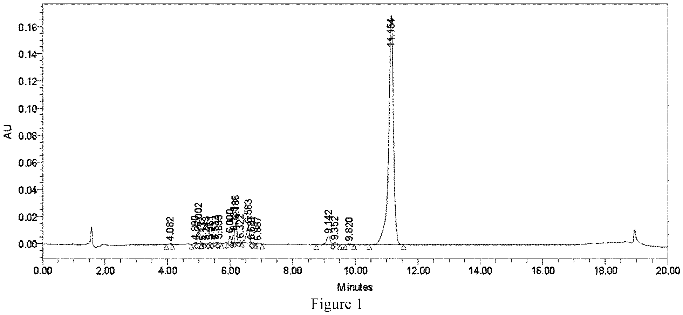

HPLC spectrum of Plinabulin formulation is shown in Figure 1.

### Example 2 Preparation of Impurity of Plinabulin

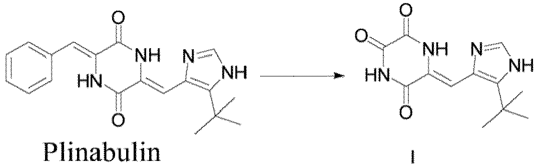

1.9482g of Plinabulin API was weighed and added into a 500 mL Erlenmeyer flask, and completely dissolved by adding ethanol (250 mL). 2N HCl (8 mL) was then added, and the API precipitated after hydrochloric acid was added. An appropriate amount of DMF was added in order to assist dissolution, and a balloon was used to seal the mouth of flask (to avoid acid evaporation). The flask was placed in an oven at 60°C, and tested every other day. pH test paper was used to detect the pH. If pH is lower than 3, an appropriate amount of acid was added till enough impurity was detected (the purity of the detected impurity of Plinabulin is no less than 5% by Plinabulin API related substances method), which then was separated and purified.

The separation and purification method are as follows:

### Reversed phase preparation liquid phase parameters:

Instrument: Flash liquid phase preparation instrument
Preparative column: C18 column
Mobile phase: A: 10 mM NH₄HCO₃ in water B: ACN
Wavelength: 214&360 nm
Flow rate: 60 mL/min
Gradients:

| Time (min) | A% | B% |
|---|---|---|
| 0 | 77 | 23 |
| 4 | 77 | 23 |
| 7.2 | 74 | 26 |
| 8.6 | 25 | 26 |
| 16.4 | 5 | 32 |
| 16.5 | 2 | 98 |
| 23 | 2 | 98 |

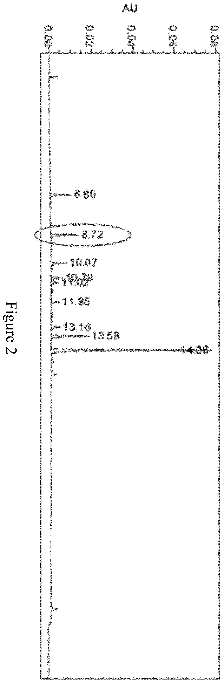

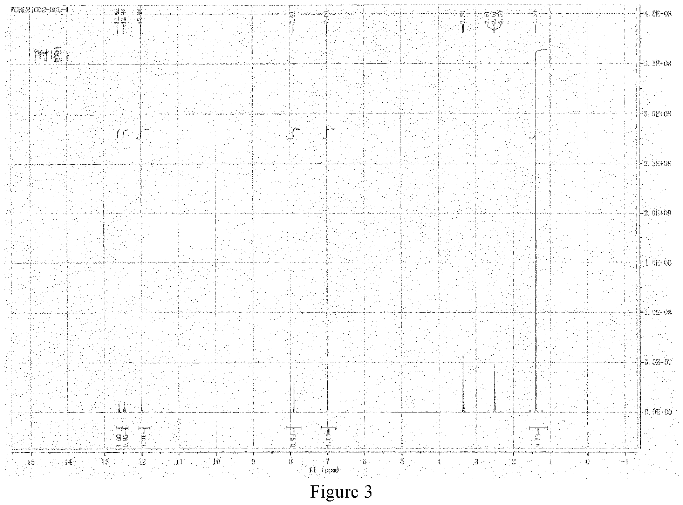

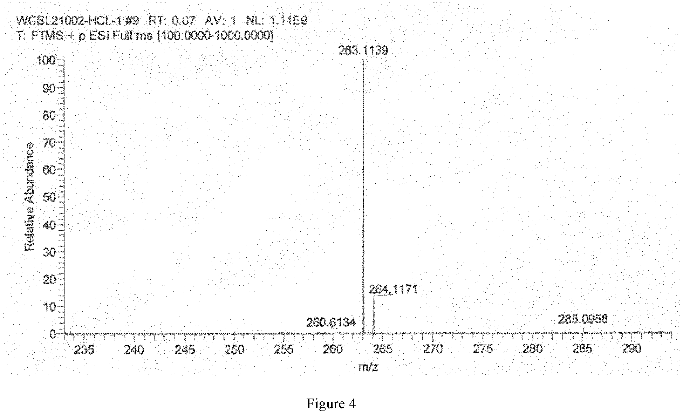

After preparation, a yellow solid precipitated from the receiving liquid, which was filtered and the residue and filtrate were detected respectively. The purity of the residue was high and it was confirmed to be the target product. A total of 58 mg of products were prepared, with a purity of 99.83% and a molecular ion peak [M+H] at 263.2. The HPLC spectrum after the reaction of Plinabulin API is shown in Figure 2, the NMR spectrum and mass spectrum are shown in Figures 3 and 4, and the NMR data are shown in Table 1 below.

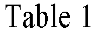

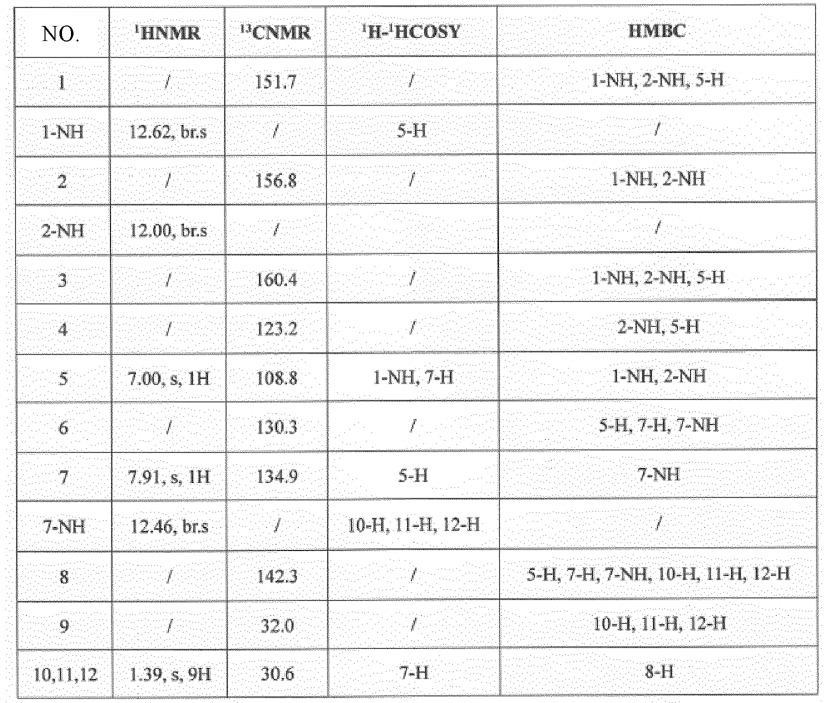

The impurity was analyzed using the same HPLC method as the analysis method of the formulation. The retention time was RT=5.016, which was consistent with the retention time of the impurity in the formulation.

### Example 3 Preparation of Impurity of Plinabulin

1 g of Plinabulin API was weighed and dissolved by adding DMF (100ml). HAc (30ml), 30% H₂O₂ (5ml) were added sequentially. The mixture was reacted in an oven at 50°C for 48 hours, and then separated and purified according to the method described in Example 2.

### Example 4 Preparation of Impurity of Plinabulin

2 g of Plinabulin API was weighed and dissolved by adding DMF (100ml). HAc (30ml), 30% H₂O₂ (20ml) were added sequentially. The mixture was reacted for 2 to 4 hours, and then separated and purified according to the method described in Example 2.

All literatures mentioned in the present invention are incorporated by reference herein, as though individually incorporated by reference. Additionally, it should be understood that after reading the above teaching, many variations and modifications may be made by the skilled in the art, and these equivalents also fall within the scope as defined by the appended claims.

## Claims

1. A compound of formula I

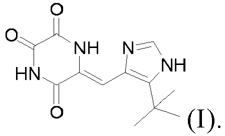

2. A composition, wherein the composition comprises i) Plinabulin and ii) the compound of formula I of claim 1.

3. The composition according to claim 2, wherein the mass ratio of Plinabulin to the compound of formula I is 1-10000:10000-1.

4. The composition of claim 2, wherein the content of Plinabulin ranges from 0.01% to 99.99%.

5. A pharmaceutical composition, wherein the pharmaceutical composition comprises i') the composition of claim 2 and ii') a pharmaceutically acceptable carrier.

6. The pharmaceutical composition of claim 5, wherein the pharmaceutical composition may be an injection, a tablet, a capsule, an aerosol, a suppository, a film, a dropping pill, an external liniment, a controlled-release type or slow-release type or nano-formulation.

7. The pharmaceutical composition of claim 5, wherein a single dose of the composition is from about 1 mg to about 300 mg.

8. A method for preparing the compound of formula I of claim 1, comprising the step: 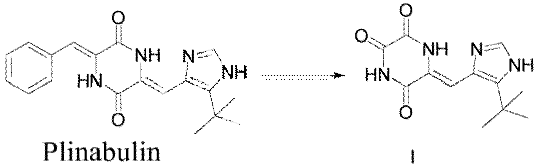
1) In an inert solvent, reacting Plinabulin with an oxidant under a neutral or acidic condition to give the compound of formula I.

9. Use of the compound of formula I of claim 1, wherein the compound is used as a standard for quality control of Plinabulin API or a preparation thereof.

10. The use of claim 7, wherein the control is achieved by using high performance liquid chromatography to determine the content of the compound of formula I in Plinabulin API or a preparation thereof that calculated by an area normalization method.
